# EUROPEAN PATENT SPECIFICATION

(11) **EP 1 587 568 B1**
(45) Date of publication and mention of the grant of the patent: **10.10.2012**
(21) Application number: 04706218.7
(22) Date of filing: 29.01.2004
(51) Int. Cl.: A61M 16/06

(54) **Nasal cannula**
Nasenkanüle
Canule nasale

(30) Priority: 30.01.2003 GB 0302196
(43) Date of publication of application: 26.10.2005
(73) Proprietor: The BOC Group Limited, The Surrey Research Park Guildford Surrey GU2 7XY (GB)
(72) Inventor: GARRETT, Michael Ernest, Woking, Surrey GU22 7XR (GB); LLOYD, Geoffrey Morgan, Sandhurst, Berkshire GU47 0FP (GB); RATHBONE, Thomas, Farnham, Surrey GU9 8DG (GB)
(74) Representative: Wickham, Michael
(86) International application number: PCT/GB2004/000352
(87) International publication number: WO 2004/067071

(56) References cited:
- EP-A- 1 334 741
- WO-A-99/24101
- US-A- 4 535 767

## Description

The present invention relates to apparatus for delivering a breathable gas mixture comprising helium and oxygen to a patient.

It is known to administer oxygen to a patient so as to facilitate their breathing.

It is also known to deliver helium/oxygen gas mixtures to a patient with breathing difficulties to reduce airway resistance and improve lung ventilation. Known methods of delivery utilise one or more cylinders of gas. Each cylinder has associated with it a two stage pressure regulator to reduce the pressure of the gas from a high storage pressure (topically in the order of 200 bar) to near atmospheric pressure. The gas flows through relatively wide diameter tubing to an administration device, for example a nasal cannula. The gas is administered to the patient at room temperature. An example of a known nasal cannula for operation of approximately atmospheric pressure is disclosed in GB-A-1 081 807. Small oxygen-conveying tubes for the purpose of conveying oxygen to a nasal cannula are disclosed in US-A-4 535 767.

It is an aim of the present invention to provide an improved apparatus for delivering a breathable gas mixture including helium to a patient which does not require plural stage gas regulation.

According to the present invention there is provided apparatus for administering to a patient a breathable gas mixture comprising helium and oxygen, including means for supplying the breathable gas mixture and a nasal cannula, wherein the nasal cannula consists of a length of high pressure narrow bore tubing having a proximal end region for connection to the high pressure means and a distal end region connected to at least one nasal administration device, and wherein the nasal administration device or the distal end region of the tubing has at least one orifice for the expansion of the breathable gas mixture, characterised in that the connection of the nasal cannula to the supply means is a high pressure connection of a pressure in the range of 100 to 300 bar.

The apparatus according to the invention offer the following advantages. First, the requirement for plural stage gas regulation to reduce the supply pressure to near atmospheric pressure is eliminated. Typically, a single stage regulator is used to supply the breathable gas at a pressure in the range of 100 to 300 bar. At such high supply pressures, the cannula tubing need only have a narrow bore. Therefore, unsightly wide bore tubing is avoided.

The breathable gas mixture is preferably a helium-oxygen mixture containing from, say, 20 to 30% by volume of oxygen (and hence 70 to 80% by volume of helium). Every gas has a Joule-Thomson inversion temperature. Above the inversion temperature throttling of the gas through an expansion orifice leads to its heating. Below the inversion temperature such throttling of the gas leads to its cooling. At ambient temperature oxygen is cooled on being expanded, but helium is warmed. The pressure at which the cannula receives the breathable gas and the composition of the mixture may thus be selected to give a desired degree of warming to the gas mixture on expansion through the said orifice. Indeed, an inhaled gas at close to the ideal tracheal temperature of the human body can be formed. Such a temperature facilitates a patient's breathing.

The breathable mixture of helium and oxygen may be stored at the desired high pressure in a gas cylinder.

The high-pressure tubing may be coiled.

The high-pressure tubing may be of a ductile metal or alloy, for example a cupro-nickel alloy.

The nasal administration device may be a device defining a nasal prong or pair of nasal prongs.

An embodiment of the invention will now be described by way of example, reference being made to the Figures of the accompanying diagrammatic drawings in which:
Figure 1 is a schematic view of an apparatus for delivering a breathable gas mixture under pressure to a patient according to the present invention;
Figures 2a and 2b are schematic details of a coiled high pressure line forming part of the apparatus of Figure 1; and
Figure 3 is a schematic detail of an end of a coiled high pressure line terminating at a tubular nasal prong forming part of the nasal cannula forming part of the apparatus of Figure 1.

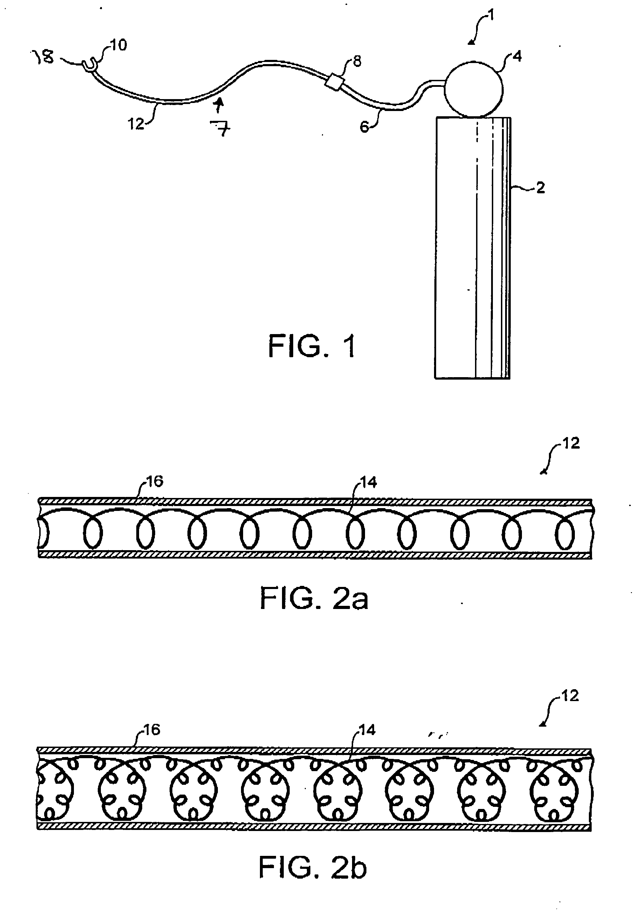

As shown in Figure 1, an apparatus 1 for delivering a breathable gas mixture including helium under pressure to a patient comprises a source of the gas mixture in the form of a cylinder 2 to which is mounted a single stage gas regulator 4 in a manner known per se. Preferably, the mixture contains 28% by volume helium, the remainder being oxygen.

A high pressure flexible braided hose 6 extends from the regulator 4 to a junction 8. A nasal cannula 7 is connected at its proximal end to the junction 8. The cannula 7 comprises a coiled high pressure tubing 12 and a nasal gas administration device 10.

Referring also to Figures 2a, 2b the coiled high pressure tubing 12 comprises a coiled high pressure cupro-nickel tube 14 having an internal diameter of between 10 and 15 thousandths of an inch enclosed within a tubular flexible protector sheath16.

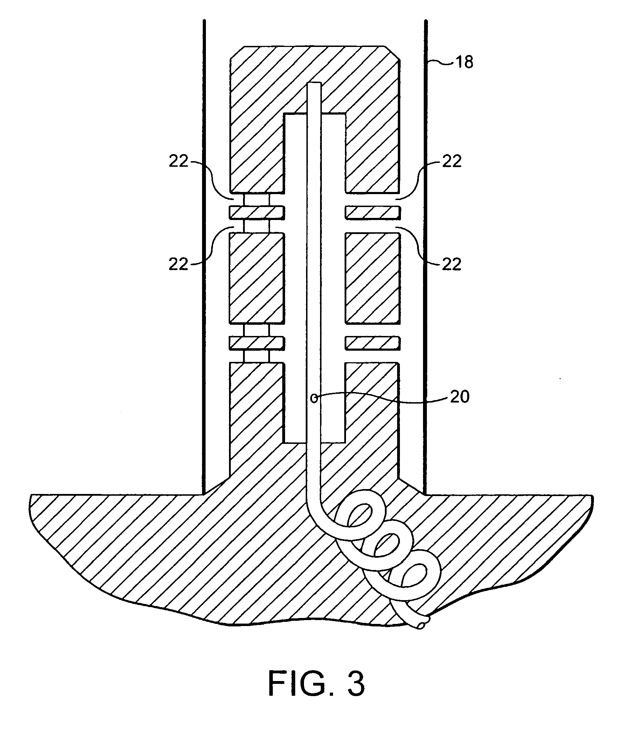

The coiled high pressure tubing 12 terminates at its distal end region in a tubular nasal prong or prongs 18 (see Figure 3) forming part of the nasal cannula 10 where it is formed with an expansion orifice 20. The tubular nasal prong 18 is formed with a plurality of perforations 22 which allow the flow of the gas mixture from the cannula 10 in to the nasal passages of a patient.

In use, the gas mixture under high pressure leaves the cylinder 2 and passes through the regulator 4. The pressure at the outlet of the regulator 4 is preferably in the range 100 bar to 300 bar. The gas mixture passes via the high pressure flexible braided hose 6 to the cannula 7..

The gas mixture leaves the coiled high pressure tubing 12 of the cannula 7 through the expansion orifice 20 and is thereby expanded from the pressure in the range of 100 bar to 300 bar to a pressure that is approximately atmospheric pressure. The atmospheric pressure gas passes through the perforations 22 for passage in to the nasal cavities of a patient.

A particular advantage of the embodiment described above is that if a mixture of oxygen and helium is expanded the combined Joule Thomson effect of expanding both helium and oxygen is one of heating. The gas is thus raised in temperature by a few degrees Celsius. In the preferred mixture of 28% helium in oxygen the temperature of gas can be raised approximately 1.4°C above ambient yielding an inhaled gas which is close to the ideal tracheal temperature of the body. Thus the patient is provided with a slightly warmed gas with enhanced oxygen and easy breathability and the pressure of the gas mixture precludes any possibility of blockage by bodily fluids.

As modelled with HYSYS v 3.0.1 software using Peng Robinson with Lee-Kessler mixing rules a 28% by volume oxygen, 72% by volume helium mixture shows the following rise in temperature following an expansion to atmospheric pressure when the mixture is received at 20°C for expansion.

| | | | |
|---|---|---|---|
| Upstream pressure (Bar) | 300 | 200 | 100 |
| Joule Thomson temperature rise (K) | 13.6 | 8.3 | 3.6 |

## Claims

1. Apparatus for administering to a patient a breathable gas mixture comprising helium and oxygen, including means (2,4,6) for supplying the breathable gas mixture and a nasal cannula (7), wherein the nasal cannula (7) consists of a length of high pressure narrow bore tubing (12) having a proximal end region for connection (at 8) to the high pressure means and a distal end region connected to at least one nasal administration device (10), and wherein the nasal administration device (10) or the distal end region of the tubing (12) has at least one orifice (29) for the expansion of the breathable gas mixture, **characterised in that** the connection (8) of the nasal cannula (7) to the supply means (2,4,6) is a high pressure connection at a pressure in the range of 100 to 300 bar.

2. Apparatus according to claim 1, wherein the high pressure narrow bore tubing (12) is coiled.

3. Apparatus according to claim 1 or claim 2, wherein the high pressure narrow bore tubing (12) is of a ductile metal or alloy.

4. Apparatus according to claim 3, wherein the alloy is a cupro-nickel alloy.

5. Apparatus according to any one of the preceding claims, wherein the high pressure narrow bore tubing (12) is surrounded by a protective sheath (16).

6. Apparatus according to any one of the preceding claims, in which the nasal administration device (10) defines a nasal prong or a pair of nasal prongs (18).

7. Apparatus according to any one of the preceding claims, wherein the said means (2,4,6) includes a gas cylinder (2) in which the breathable gas mixture is stored under pressure.

8. Apparatus according to claim 7, wherein the stored breathable gas mixture contains from 70 to 80% by volume of helium and from 20 to 30% by volume of oxygen.

9. Apparatus according to claim 7 or claim 8, wherein the stored breathable gas mixture is stored in the cylinder at a pressure in the range of 10Q bar to 300 bar.

10. Apparatus according to any one of claims 7 to 9, wherein the stored breathable gas mixture contains 72% by volume of helium, balance oxygen.

## Patentansprüche

1. Gerät zur Verabreichung eines Helium und Sauerstoff umfassenden Atemgasgemisches an einen Patienten, mit Mitteln (2, 4, 6) zu Zuführung des Atemgasgemisches und einer Nasenkanüle (7), worin die Nasenkanüle (7) aus einem Stück Hochdruckschlauch (12) mit engen Bohrungen mit einer proximalen Endregion zum Anschließen (bei 8) des Hochdruckmittels und einer distalen Endregion, die zumindest mit einer Nasenverabreichungsvorrichtung (10) verbunden ist, besteht, und worin die Nasenverabreichungsvorrichtung (10) oder die distale Endregion des Schlauchs (12) zumindest eine Öffnung (29) zur Ausdehnung des Atemgasgemisches aufweist, **dadurch gekennzeichnet, dass** die Verbindung (8) der Nasenkanüle (7) mit dem Zuführungsmittel (2, 4, 6) eine Hochdruckverbindung mit einem Druck im Bereich von 100 bis 300 bar ist.

2. Gerät nach Anspruch 1, worin der Hochdruckschlauch (12) mit engen Bohrungen aufgerollt ist.

3. Gerät nach Anspruch 1 oder Anspruch 2, worin der Hochdruckschlauch (12) mit engen Bohrungen aus einem formbaren Metall oder einer Legierung besteht.

4. Gerät nach Anspruch 3, worin die Legierung eine Kupfernickellegierung ist.

5. Gerät nach einem der vorhergehenden Ansprüche, worin der Hochdruckschlauch (12) mit engen Bohrungen von einer Schutzhülle (16) umgeben ist.

6. Gerät nach einem der vorhergehenden Absprüche, in dem die Nasenverabreichungsvorrichtung (10) eine Nasengabel oder ein Nasengabelpaar (18) definiert.

7. Gerät nach einem der vorhergehenden Ansprüche, worin das Mittel (2, 4, 6) einen Gaszylinder (2) aufweist, in dem das Atemgasgemisch unter Druck aufbewahrt wird.

8. Gerät nach Anspruch 7, worin das aufbewahrte Atemgasgemisch 70 bis 80 Volumen-% Helium und 20 bis 30 Volumen-% Sauerstoff enthält.

9. Gerät nach Anspruch 7 oder Anspruch 8, worin das aufbewahrte Atemgasgemisch im Zylinder mit einem Druck im Bereich von 100 bar bis 300 bar aufbewahrt wird.

10. Gerät nach einem der Ansprüche 7 bis 9, worin das aufbewahrte Atemgasgemisch 72 Volumen-% Helium enthält und der Rest Sauerstoff ist.

## Revendications

1. Dispositif pour administrer à un patient un mélange de gaz respirable comprenant de l'hélium et de l'oxygène, comportant des moyens (2, 4, 6) pour fournir le mélange de gaz respirable et une canule nasale (7), dans lequel la canule nasale (7) se compose d'une longueur de tuyau à canal étroit à haute pression (12) présentant une région d'extrémité proximale pour la connexion (en 8) aux moyens de haute pression et une région d'extrémité distale connectée à au moins un dispositif d'administration nasale (10), et dans lequel le dispositif d'administration nasale (10) ou la région d'extrémité distale du tuyau (12) présente au moins un orifice (29) destiné à la dilatation du mélange de gaz respirable, **caractérisé en ce que** la connexion (8) de la canule nasale (7) aux moyens d'alimentation (2, 4, 6) est une connexion à haute pression à une pression comprise dans la plage de 100 à 300 bars.

2. Dispositif selon la revendication 1, dans lequel le tuyau à canal étroit à haute pression (12) est bobiné.

3. Dispositif selon la revendication 1 ou la revendication 2, dans lequel le tuyau à canal étroit à haute pression (12) est en métal ou en alliage ductile.

4. Dispositif selon la revendication 3, dans lequel l'alliage est un alliage de cupro-nickel.

5. Dispositif selon l'une quelconque des revendications précédentes, dans lequel le tuyau à canal étroit à haute pression (12) est entouré d'une gaine de protection (16).

6. Dispositif selon l'une quelconque des revendications précédentes, dans lequel le dispositif d'administration nasale (10) définit une fourche nasale ou une paire de fourches nasales (18).

7. Dispositif selon l'une quelconque des revendications précédentes, dans lequel lesdits moyens (2, 4, 6) comprennent un cylindre de gaz (2) dans lequel le mélange de gaz respirable est stocké sous pression.

8. Dispositif selon la revendication 7, dans lequel le mélange de gaz respirable stocké contient de 70 à 80 % en volume d'hélium et de 20 à 30 % en volume d'oxygène.

9. Dispositif selon la revendication 7 ou la revendication 8, dans lequel le mélange de gaz respirable stocké est stocké dans le cylindre à une pression comprise dans la plage de 100 bars à 300 bars.

10. Dispositif selon l'une quelconque des revendications 7 à 9, dans lequel le mélange de gaz respirable stocké contient 72 % en volume d'hélium, le reste étant de l'oxygène.
